# EUROPEAN PATENT APPLICATION

(11) **EP 2 460 519 A1**
(43) Date of publication of application: **06.06.2012**
(21) Application number: 10382330.8
(22) Date of filing: 03.12.2010
(51) Int. Cl.: A61K 31/4152, A61K 31/4155, A61K 31/4523, A61K 31/4725, A61K 31/496, A61K 31/5377, A61P 29/00

(54) **Use of sigma ligands in bone cancer pain**

(71) Applicant: Laboratorios del. Dr. Esteve, S.A., 08041 Barcelona (ES)
(72) Inventor: Zamanillo Castanedo, Daniel, E-08041 Barcelona (ES); Vela Hernández, José Miguel, E-08028 Barcelona (ES); Plata Salaman, Carlos, E-08950 - Esplugues de Llobregat Barcelona (ES)
(74) Representative: ABG Patentes, S.L.

(57) **Abstract**

The invention refers to the use of a sigma ligand, particularly a sigma ligand of formula (I) to prevent and/or treat pain associated to bone cancer.

## Description

### FIELD OF THE INVENTION

The present invention relates to sigma receptor ligands, more particularly to some indazole derivatives, and pharmaceutical compositions comprising them, for use in therapy and/or prophylaxis of pain associated to bone cancer.

### BACKGROUND

Bone cancer pain is a devastating manifestation of metastatic cancer. Unfortunatelly, current therapies can be ineffective, and when they are effective, the duration of the patient's survival typically exceeds the duration of the pain relief. New, mechanistically based therapies are desperately needed.

Patients with malignant tumors that have metastasized to bone are frequently confronted with poor quality of life. Skeletal complications as sequelae of metastatic disease manifest themselves in ∼70% of patients with advanced breast or prostatic carcinoma. Skeletal metastases are discovered in >90% of patients who die of breast or prostate carcinoma. Bone cancer pain is one of the most common symptoms presented by patients with cancer. Metastatic breast and prostate carcinomas are principal contributors to the prevalence of cancer-induced bone pain. Thus the pain from bone cancer also represents the most common pain in human patients with advanced cancer, because most common tumors including breast, prostate and lung cancers have a remarkable propensity to metastasize to bone.

Human cancer patients who are in advanced stages of the disease, particularly those with bone metastasis, report that they experience significant pain and pain intensity appears to be related to the degree of bone destruction.

Cancer patients with skeletal involvement often suffer from fractures, hypercalcemia, spinal cord compression and severe pain, all of which, as mentioned, contribute to an increased morbidity and decreased quality of life.

Pain originating from skeletal metastases usually increases in magnitude over the evolution of the disease and is commonly divided into two categories: ongoing pain and breakthrough or incident pain.

Ongoing pain which is usually the first symptom of bone cancer, begins as a dull, constant, throbbing pain that increases in intensity with time and is exacerbated by use of involved portions of skeleton.

With increased bone destruction and time, the pain intensifies and then intermittent episodes of extreme pain can occur spontaneously or, more commonly, after weigh-bearing or movement of the affected limb. This pain is referred to as "breakthrough or incident pain".

Of these two types of pain, breakthrough pain is more difficult to control as, for instance, the dose of opioids needed to control this pain are frequently higher than that needed to control ongoing pain and thus are accompanied by significant unwanted side effects as commented after.

Mechanical allodynia is the painful perception of mechanical stimuli that are not normally perceived as noxious. With this bone cancer pain, this acute form of movement-evoked pain can be generated by modest limb use, coughing or turning in bed and has diminished responsiveness to conventional therapeutics.

Pain intensity varies among cancer patients and is dependent on a patient's pain sensitivity, the type of cancer and the tumor location. Cancer treatment guidelines provided by the World Health Organization (WHO) have been used in oncology and pain treatment clinics. Treatment of human cancer patients include the use of opioids, non-steroidal anti-inflammatory drugs (NSAIDs), corticosteroids, local anhestetics, antidepressants and anticonvulsants either alone or in combination.

Chronic pain unresponsive to anti-inflammatory agents, chemotherapy, radiotherapy, surgery and/or bisphosphonates is typically combated with strong pain medications. Opioid management of advanced bone cancer pain is common and effective for pain relief. Unfortunately, opioid doses required to attenuate bone pain (120 mg/kg daily) can produce undesirable side effects, such as confusion, respiratory depression, somnolence and constipation. These side effects can severely diminish overall quality of life. Within 4 weeks after seeing their physician, 73% of terminally ill patients receiving opioid treatment reported pain that was moderate to severe and 40% of those patients with severe pain requested an increase in opioid treatment.

The escalating doses of opioids required to control the pain may reflect either an evolving opioid tolerance or an increase in the severity of pain. Although both mechanisms are probably involved in the escalating dose of morphine required for this pain, the ability to notably reduce opioid requirements by the use of palliative therapies suggest that the high opioid doses are at least partially a reflection of the intensity associated with the bone cancer pain state.

Because opioid do not directly target the source of pain but act systematically via the central nervous system, the negative repercussions to organ systems can contribute significantly to poor quality of life (Clin. Cancer Res., 2006, 12(20 Supplm., 6231s-6235s; Compar. Med., 2008, 58(3), 220-233; J. Pain and Symp. Manag., 2005, 29(55), S32-S46).

Animal models of cancer pain can be divided into the following five categories: bone cancer pain models, non-bone cancer pain models, cancer invasion pain models, cancer chemotherapeutic-induced peripheral neuropathy models and spontaneous occurring cancer pain models. Specifically, bone cancer pain has been studied, among others, using a rat breast carcinoma model (MRMT-1), murine fibrosarcoma (2472), murine breast carcinomas (4T1), hepatocellular carcinomas (HCa-1) and murine melanoma (B16).

Use of these models has revealed numerous features associated with pain-related behaviors and has provided insight into neurochemical and neurophysiological mechanisms that underlie cancer pain. Many of the features observed in these animal models are shared by human cancer patients that experiences tumor pain including bone destruction, primary afferent neuron sensitization and reorganization and development of central sensitization in the spinal cord.

As previously mentioned, a major problem in dealing with advanced bone cancer pain is that whereas opioids may be able to control ongoing pain, these same doses are frequently insufficient to block movement-evoked breakthrough pain.

As data present in the art suggests that bone cancer pain may have both an inflammatory and a neuropathic component and even though significant progress has been made recently in experimental models that examine potential new therapies, clearly more effective treatments with greater efficacy versus inflammatory and neuropathic component for bone cancer pain are needed due to that the goal of pain management is not only to alleviate the pain, but also to maintain the patient's physiological and psychological well-being.

### BRIEF DESCRIPTION OF THE INVENTION

The present invention relates to the use of a sigma ligand as adjuvant in the therapy of bone cancer pain. This benefit of the invention is more evident when the sigma ligand is specifically a sigma-1 receptor antagonist, preferably in the form of a (neutral) antagonist, an inverse agonist or a partial antagonist.

Therefore, one aspect of the present invention relates to a sigma ligand for use in the prevention and/or treatment of pain associated to bone cancer.

In a preferred embodiment, said sigma ligand has the general formula (I): 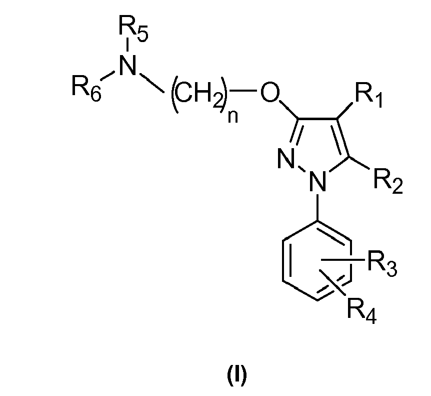 wherein
**R₁** is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted non-aromatic heterocyclyl, substituted or unsubstituted aromatic heterocyclyl, substituted or unsubstituted heterocyclylalkyl, -COR₈, -C(O)OR₈, - C(O)NR₈R₉, -CH=NR₈, -CN, -OR₈, -OC(O)R₈, -S(O)ₜ-R₈, -NR₈R₉, -NR₈C(O)R₉, - NO₂, -N=CR₈R₉, and halogen;
**R₂** is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈, -C(O)NR₈R₉, -CH=NR₈, -CN, - OR₈, -OC(O)R₈, -S(O)ₜ-R₈, -NR₈R₉, -NR₈C(O)R₉, -NO₂, -N=CR₈R₉, and halogen;
**R₃** and **R₄** are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈, - C(O)NR₈R₉, -CH=NR₈, -CN, -OR₈, -OC(O)R₈, -S(O)ₜ-R₈, -NR₈R₉, -NR₈C(O)R₉, - NO₂, -N=CR₈R₉, and halogen, or together they form an optionally substituted fused ring system;
**R₅** and **R₆** are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈, - C(O)NR₈R₉, -CH=NR₈, -CN, -OR₈, -OC(O)R₈, -S(O)ₜ-R₈, -NR₈R₉, -NR₈C(O)R₉, - NO₂, -N=CR₈R₉, and halogen, or together form, with the nitrogen atom to which they are attached, a substituted or unsubstituted, aromatic or non-aromatic heterocyclyl group;
**n** is selected from 1, 2, 3, 4, 5, 6, 7 and 8;
**t is** 1,2 or 3;
**R₈** and **R₉** are each independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted, aromatic or non-aromatic heterocyclyl, substituted or unsubstituted alkoxy, substituted or unsubstituted aryloxy, and halogen;
or a pharmaceutically acceptable salt, isomer, prodrug or solvate thereof.

Another aspect of this invention refers to the use of sigma ligand as defined above for the manufacture of a medicament for the prevention and/or treatment of pain associated to bone cancer.

Another aspect of the invention is a method of treatment of a patient suffering pain associated to a bone cancer, or likely to suffer pain as a result of bone cancer, which comprises administering to the patient in need of such a treatment or prophylaxis a therapeutically effective amount of a sigma ligand as defined above.

Another aspect of the invention refers to a combination of at least one sigma ligand as defined above and at least one opioid or opiate compound for simultaneous, separate or sequential administration, for use in the prevention and/or treatment of pain associated to a bone cancer.

These aspects and preferred embodiments thereof are additionally also defined in the claims.

### BRIEF DESCRIPTION OF THE FIGURES

**Figure 1****:** Von Frey test. Determination of the optimal dose level of morphine for the second phase (Phase 2).
**Figure 2****:** Von Frey test. Synergistic effect of morphine (1.25 mg/kg) and example 1 (40 mg/kg) in Phase 2.

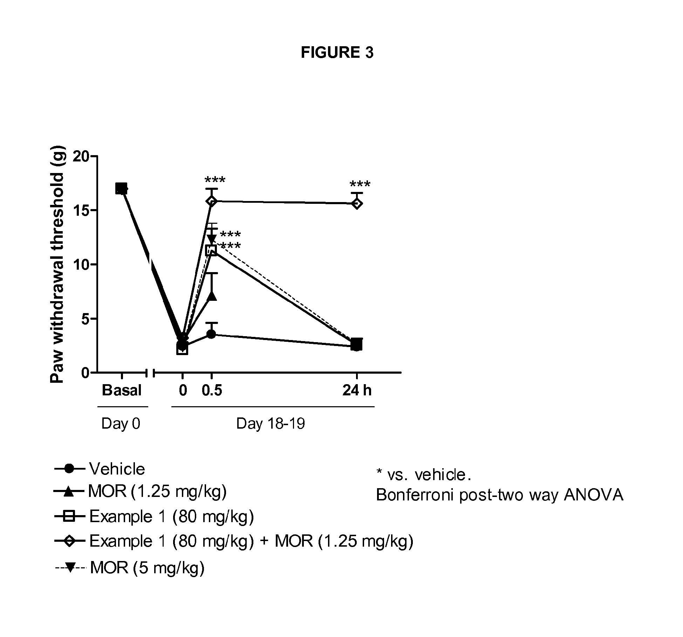

**Figure 3****:** Von Frey test. Synergistic effect of morphine (1.25 mg/kg) and example 1 (80 mg/kg) in Phase 2.

### DETAILED DESCRIPTION OF THE INVENTION

In the context of the present invention, the following terms have the meaning detailed below.

"Alkyl" refers to a straight or branched hydrocarbon chain radical consisting of 1 to 12 carbon atoms, containing no unsaturation, and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as aryl, halo, hydroxy, alkoxy, carboxy, cyano, carbonyl, acyl, alkoxycarbonyl, amino, nitro, mercapto, alkylthio, etc. Preferred alkyl radicals have from 1 to 6 carbon atoms. If substituted by aryl, it corresponds to an "Arylalkyl" radical, such as benzyl or phenethyl. If substituted by heterocyclyl, it corresponds to a "Heterocyclylalkyl" radical.

"Alkenyl" refers to a straight or branched hydrocarbon chain radical consisting of 2 to 12 carbon atoms, containing at least one unsaturation, and which is attached to the rest of the molecule by a single bond. Alkenill radicals may be optionally substituted by one or more substituents such as aryl, halo, hydroxy, alkoxy, carboxy, cyano, carbonyl, acyl, alkoxycarbonyl, amino, nitro, mercapto, alkylthio, etc. Preferred alkenyl radicals have from 2 to 6 carbon atoms.

"Cycloalkyl" refers to a stable 3-to 10-membered monocyclic or bicyclic radical which is saturated or partially saturated, and which consist solely of carbon and hydrogen atoms, such as cyclohexyl or adamantyl. Unless otherwise stated specifically in the specification, the term "cycloalkyl" is meant to include cycloalkyl radicals which are optionally substituted by one or more substituents such as alkyl, halo, hydroxy, amino, cyano, nitro, alkoxy, carboxy, alkoxycarbonyl, etc.

"Aryl" refers to single and multiple aromatic ring radicals, including multiple ring radicals that contain separate and/or fused aryl groups. Typical aryl groups contain from 1 to 3 separated or fused rings and from 6 to about 18 carbon ring atoms, such as phenyl, naphthyl, indenyl, fenanthryl or anthracyl radical. The aryl radical may be optionally substituted by one or more substituents such as hydroxy, mercapto, halo, alkyl, phenyl, alkoxy, haloalkyl, nitro, cyano, dialkylamino, aminoalkyl, acyl, alkoxycarbonyl, etc.

"Heterocyclyl" refers to a stable 3-to 15 membered ring radical which consists of carbon atoms and from one to five heteroatoms selected from the group consisting of nitrogen, oxygen, and sulfur, preferably a 4-to 8-membered ring with one or more heteroatoms, more preferably a 5-or 6-membered ring with one or more heteroatoms. It may be aromatic or not aromatic. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran, coumarine, morpholine; pyrrole, pyrazole, oxazole, isoxazole, triazole, imidazole, etc.

"Alkoxy" refers to a radical of the formula -ORₐ where Rₐ is an alkyl radical as defined above, e. g., methoxy, ethoxy, propoxy, etc.

"Amino" refers to a radical of the formula -NH₂, -NHRₐ or -NRₐR_{b}, optionally quaternized, e.g., methylamino, ethylamino, dimethylamino, diethylamino, propylamino, etc.

"Halogen", "halo" or "hal" refers to bromo, chloro, iodo or fluoro.

References herein to substituted groups in the compounds of the present invention refer to the specified moiety that may be substituted at one or more available positions by one or more suitable groups, e. g., halogen such as fluoro, chloro, bromo and iodo; cyano; hydroxyl; nitro; azido; alkanoyl such as a C₁₋₆ alkanoyl group such as acyl and the like; carboxamido; alkyl groups including those groups having 1 to about 12 carbon atoms or from 1 to about 6 carbon atoms and more preferably 1-3 carbon atoms; alkenyl and alkynyl groups including groups having one or more unsaturated linkages and from 2 to about 12 carbon or from 2 to about 6 carbon atoms; alkoxy groups having one or more oxygen linkages and from 1 to about 12 carbon atoms or 1 to about 6 carbon atoms; aryloxy such as phenoxy; alkylthio groups including those moieties having one or more thioether linkages and from 1 to about 12 carbon atoms or from 1 to about 6 carbon atoms; alkylsulfinyl groups including those moieties having one or more sulfinyl linkages and from 1 to about 12 carbon atoms or from 1 to about 6 carbon atoms; alkylsulfonyl groups including those moieties having one or more sulfonyl linkages and from 1 to about 12 carbon atoms or from 1 to about 6 carbon atoms; aminoalkyl groups such as groups having one or more N atoms and from 1 to about 12 carbon atoms or from 1 to about 6 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

The term "salt" must be understood as any form of an active compound used in accordance with this invention in which said compound is in ionic form or is charged and coupled to a counter-ion (a cation or anion) or is in solution. This definition also includes quaternary ammonium salts and complexes of the active molecule with other molecules and ions, particularly, complexes formed via ionic interactions. The definition includes in particular physiologically acceptable salts; this term must be understood as equivalent to "pharmacologically acceptable salts" or "pharmaceutically acceptable salts".

The term "pharmaceutically acceptable salts" in the context of this invention means any salt that is tolerated physiologically (normally meaning that it is not toxic, particularly, as a result of the counter-ion) when used in an appropriate manner for a treatment, applied or used, particularly, in humans and/or mammals. These physiologically acceptable salts may be formed with cations or bases and, in the context of this invention, are understood to be salts formed by at least one compound used in accordance with the invention -normally an acid (deprotonated)- such as an anion, particularly when used on humans and/or mammals. These physiologically acceptable salts may also be formed with anions or acids and, in the context of this invention, are understood as being salts formed by at least one compound used in accordance with the invention - normally protonated, for example in nitrogen - such as a cation and at least one physiologically tolerated anion, particularly when used on humans and/or mammals. This definition specifically includes in the context of this invention a salt formed by a physiologically tolerated acid, i.e. salts of a specific active compound with physiologically tolerated organic or inorganic acids - particularly when used on humans and/or mammals. Examples of this type of salts are those formed with: hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid.

The term "solvate" in accordance with this invention should be understood as meaning any form of the active compound in accordance with the invention in which said compound is bonded by a non-covalent bond to another molecule (normally a polar solvent), including especially hydrates and alcoholates, like for example, methanolate. A preferred solvate is the hydrate.

Any compound that is a prodrug of a sigma ligand, in particular a prodrug of a compound of formula (I) is also within the scope of the invention. The term "prodrug" is used in its broadest sense and encompasses those derivatives that are converted in vivo to the compounds of the invention. Examples of prodrugs include, but are not limited to, derivatives and metabolites of the compounds of formula I that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger "Medicinal Chemistry and Drug Discovery 6th ed. (Donald J. Abraham ed., 2001, Wiley), "Design and Applications of Prodrugs" (H. Bundgaard ed., 1985, Harwood Academic Publishers) and Krogsgaard-Larsen et al. "Textbook of Drug design and Discovery" Taylor & Francis (April 2002).

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

Furthermore, any compound referred to herein may exist as tautomers. Specifically, the term tautomer refers to one of two or more structural isomers of a compound that exist in equilibrium and are readily converted from one isomeric form to another. Common tautomeric pairs are amine-imine, amide-imidic acid, keto-enol, lactam-lactim, etc.

Unless otherwise stated, the compounds of the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by ¹³C- or ¹⁴C-enriched carbon, or the replacement of at least one nitrogen by ¹⁵N-enriched nitrogen are within the scope of this invention.

The sigma ligands, in particular the compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

As noted previously, the term "pharmaceutically acceptable salts, solvates, prodrugs" refers to any salt, solvate, or any other compound which, upon administration to the recipient is capable of providing (directly or indirectly) a compound as described herein. However, it will be appreciated that non-pharmaceutically acceptable salts, solvates and prodrugs also fall within the scope of the invention since those may be useful in the preparation of pharmaceutically acceptable salts, solvates and prodrugs. The preparation of salts, solvates and prodrugs can be carried out by methods known in the art.

As used herein, the terms "treat", "treating" and "treatment" include the eradication, removal, reversion, alleviation, modification, or control of pain associated to bone cancer.

As used herein, the terms "sigma ligand" or "sigma receptor ligand" refer to any compound binding to the sigma receptor. As stated previously, the sigma ligand is preferebly a sigma receptor antagonist in the form of a (neutral) antagonist, an inverse agonist or a partial antagonist.

An "agonist" is defined as a compound that binds to a receptor and has an intrinsic effect, and thus, increases the basal activity of a receptor when it contacts the receptor. An "antagonist" is defined as a compound that competes with an agonist or inverse agonist for binding to a receptor, thereby blocking the action of an agonist or inverse agonist on the receptor. However, an antagonist (also known as a "neutral" antagonist) has no effect on constitutive receptor activity. Antagonists mediate their effects by binding to the active site or to allosteric sites on receptors, or they may interact at unique binding sites not normally involved in the biological regulation of the receptor's activity. Antagonist activity may be reversible or irreversible depending on the longevity of the antagonist-receptor complex, which, in turn, depends on the nature of antagonist receptor binding.

A "partial antagonist" is defined as a compound that binds to the receptor and generates an antagonist response; however, a partial antagonist does not generate the full antagonist response. Partial antagonists are weak antagonists, thereby blocking partially the action of an agonist or inverse agonist on the receptor.

An "inverse agonist" is defined as a compound that produces an effect opposite to that of the agonist by occupying the same receptor and, thus, decreases the basal activity of a receptor (i.e., signalling mediated by the receptor). Such compounds are also known as negative antagonists. An inverse agonist is a ligand for a receptor that causes the receptor to adopt an inactive state relative to a basal state occurring in the absence of any ligand. Thus, while an antagonist can inhibit the activity of an agonist, an inverse agonist is a ligand that can alter the conformation of the receptor in the absence of an agonist.

"The sigma receptor/s" as used in this application is/are well known and defined using the following citation: "this binding site represents a typical protein different from opioid, NMDA, dopaminergic, and other known neurotransmitter or hormone receptor families" (G. Ronsisvalle et al. Pure Appl. Chem. 73, 1499-1509 (2001)). Pharmacological data based on ligand binding studies, anatomical distribution and biochemical features distinguish at least two subtypes of σ receptors (R. Quiron et al., Trends Pharmacol. Sci. 13, 85-86 (1992); M.L.Leitner, Eur. J. Pharmacol. 259, 65-69 (1994); S.B. Hellewell and W.D. Bowen; Brain Res. 527, 244-253 (1990)) (G. Ronsisvalle et al. Pure Appl. Chem. 73, 1499-1509 (2001)). The protein sequences of the sigma receptors (Sigma 1 (σ1) and Sigma 2 (σ2)) are known in the art (e.g. Prasad, P.D. et al., J. Neurochem. 70 (2), 443-451 (1998)). They show a very high affinity to various analgesics (e.g. pentazocine).

"Compound/s binding to the sigma receptor" or "sigma ligand" as used in this application is/are defined as a compound having an IC₅₀ value of ≤ 5000 nM, more preferably ≤ 1000 nM, more preferably ≤ 500 nM on the sigma receptor. More preferably, the IC₅₀ value is ≤ 250 nM. More preferably, the IC₅₀ value is ≤ 100 nM. Most preferably, the IC₅₀ value is ≤ 50 nM. The half maximal inhibitory concentration (IC50) is a measure of the effectiveness of a compound in inhibiting biological or biochemical function. The IC50 is the concentration of competing ligand which displaces 50% of the specific binding of the radioligand. Additionally, the wording "Compound/s binding to the sigma receptor", as used in the present application is defined as having at least ≥50% displacement using 10 nM radioligand specific for the sigma receptor (e.g. preferably [³H]-(+) pentazocine) whereby the sigma receptor may be any sigma receptor subtype. Preferably, said compounds bind to the sigma-1 receptor subtype.

Compounds binding to the sigma receptor, generally also referred to as sigma ligands, are well known in the art. Many of them are encompassed by the "Compound/s binding to the sigma receptor" definition above. Although there are many known uses for sigma ligands, such as antipsychotic drugs, anxiolytics, antidepressants, stroke treatment, antiepileptic drugs and many other indications, including anti-migraine and general pain, there is no mention in the art of these compounds as useful for the treatment of of pain associated to a bone cancer.

Table 1 lists some sigma ligands known in the art (i.e. having an IC₅₀ ≤ 5000 nM). Some of these compounds may bind to the sigma-1 and/or to the sigma-2 receptor. These sigma ligands also include their respective salts, bases, and acids.

**Table 1**

| | |
|---|---|
| (-)-Cyanopindolol hemifumarate | Cutamesine hydrochloride |
| (-)-(1R,2S)-cis-N-[2-(3,4-Dichlorophenyl)ethyl]-2-pyrrolidinocyclohexylamine | Cyclobenzaprine HCl |
| (-)-1-[1-(3-Chlorophenyl)pyrrolidin-2-ylmethyl]-4-(2-phenylethyl)piperazine | Cycloheximide |
| (-)-Sparteine sulfate pentahydrate | Cyproheptadine HCl |
| (+)-Himbacine | Darrow Red HCl |
| (±)-1-Cyclohexyl-4-[3-(5-methoxy-1,2,3,4-tetrahydronaphthalen-1-yl)propyl]piperazine | Demecarium Bromide |
| (1 S,SR)-3-[2-(2-Adamantyl)ethyl]-1,8,8-trimethyl-3-azabicyclo[3.2.1]octane hydrochloride | Denatonium Benzoate |
| (2-Dibutylamino-Ethyl)-Carbamic Acid 2-(4-Benzofuran-2-Ylmethyl-Piperazin-1-Yl)-Ethyl Ester | Deptropine Citrate |
| (4-[1,2,3]Thiadiazol-4-Yl-Benzyl)-Carbamic Acid 1-(3-Methoxy-2-Nitro-Benzyl)-Piperidin-3-Ylmethyl Ester | Desloratadine |
| (4aalpha,8aalpha)-6-(4-Fluorophenyl)-2-(4-pyridylmethyl)-6-hydroxydecahydroisoquinoline; (4a,8a-cis)-6-(4-Fluorophenyl)-2-(pyridin-4-ylmethyl)perhydroisoquinolin-6-ol | Dexbrompheniramine Maleate |
| (4aalpha,8abeta)-2-Benzyl-6-(4-fluorophenyl)-6-hydroxydecahydroisoquinoline | Dexchlorpheniramine Maleate |
| (6aR,9R)-5-Bromo-7-methyl-N-(2-propynyl)-4,6,6a,7,8,9-hexahydroindolo[4,3-fg]quinoline-9-carboxamide | Dexfenfluramine HCl |
| (S)-(-)-N-(2-Amino-3-phenylpropyl)-2-(3,4-dichlorophenyl)-N-methylacetamide hydrochloride | Dicyclomine HCl |
| (S)-Methamphetamine HCl | Diethylpropion HCl |
| [1-(9-Ethyl-9H-Carbazol-3-Ylmethyl)-Pyrrolidin-3-Yl]-Carbamic Acid 1-(3-Benzyloxy-4-Methoxy-Benzyl)-Piperidin-3-Ylmethyl Ester | Dimethisoquin HCl |
| [1-(9-Ethyl-9H-Carbazol-3-Ylmethyl)-Pyrrolidin-3-Yl]-Carbamic Acid 2-(Tert-Butoxycarbonyl-Naphthalen-1-Ylmethyl-Amino)-Ethyl Ester | Dimetindene Maleate |
| [4-(4-Ethyl-3,5-Dimethyl-Pyrazol-1-Yl)-Phenyl]-[4-(3-Phenyl-Allyl)-Piperazin-1-Yl]-Methanone | Diphemanil Methylsulfate |
| 1-(1,2-Diphenylethyl)Piperidine Maleate, (+/-) | Diphenidol HCl |
| 1-(1,4-Ethano-1,2,3,4-tetrahydro-2-naphthylmethyl)-4-methylpiperazine hydrate; 1-(Benzobicyclo[2.2.2]octen-2-ylmethyl)-4-methylpiperazine hydrate | Diphenoxylate HCl |
| 1-(1-Adamantyl)-2-[4-(2H-naphtho[1,8-cd]isothiazol-2-ylmethyl)piperidin-1-yl]ethanone S,S-dioxide hydrochloride | Diphenylpyraline HCl |
| 1-(1-Naphthyl)Piperazine HCl | Dipropyldopamine HBr |
| 1-(2-Benzyloxyethyl)-4-(3-phenylpropyl)piperazine dihydrochloride | Doxepin HCl |
| 1-(2-Phenylethyl)piperidine oxalate | Dyclonine HCl |
| 1-(3-Chlorophenyl)Piperazine HCl | Ebastine |
| 1-(3-Chlorothien-2-yl)-2-[4-(4-fluorobenzyl)piperidin-1-yl]ethanol | Econazole Nitrate |
| 1-(4-Bromo-Benzenesulfonyl)-4-(2-Tert-Butylsulfanyl-Benzyl)-Piperazine | Epinastine HCl |
| 1-(4-Chloro-3-hydroxyphenyl)-2-[4-(4-fluorobenzyl)piperidin-1-yl]ethanol | Ethaverine HCl |
| 1-(4-Chlorophenyl)-3-(hexahydroazepin-1-ylmethyl)pyrrolidin-2-one | Ethopropazine HCl |
| 1-(4-Chlorophenyl)-3(R)-[4-(2-methoxyethyl)-1-piperazinylmethyl]pyrrolidin-2-one (-)-D-tartrate | Eticlopride HCl, S(-)- |
| 1-(4-Chlorophenyl)-3(R)-[4-(2-methoxyethyl)piperazin-1-ylmethyl]pyrrolidin-2-one dihydrochloride | Etofenamate |
| 1'-(4-Fluorobenzyl)-1,3-dihydrospiro[2-benzofuran-1,4'-piperidine] | Etonitazenyl Isothiocyanate |
| 1-(4-Fluorophenyl)-4-[4-(5-fluoro-2-pyrimidinyl)-1-piperazinyl]butan-1-ol hydrochloride | Femoxetine HCl |
| 1-(4-Fluorophenyl)-4-[4-(5-fluoropyrimidin-2-yl)piperazin-1-yl]butan-1-ol; 1-[4-(4-Fluorophenyl)-4-hydroxybutyl]-4-(5-fluoropyrimidin-2-yl)piperazine | Fenfluramine HCl |
| 1'-(4-Phenylbutyl)spiro[1,3-dihydroisobenzofuran-1,4'-piperidine] | Fenticonazole Nitrate |
| 1-(Cyclobutylmethyl)-2-[3-phenyl-2(E)-propenyl]pyrrolidine hydrochloride | Fipexide HCl |
| 1-(Cyclohexylmethyl)-3'-methoxy-5'-phenyl-4',5'-dihydro-3'H-spiro[piperidine-4,1'-pyrano[4,3-c]pyrazole] | Flavoxate HCl |
| 1-(Cyclopropylmethyl)-4-[2-(4-fluorophenyl)-2-oxoethyl]piperidine hydrobromide | Flunarizine diHCl |
| 1,4-Bis[spiro[isobenzofuran-1(3H),4'-piperidin]-1'-yl]butane | Fluoxetine Related Compound B |
| 1-[(1R,3R)-2,2-Dimethyl-3-(2-phenoxyethyl)cyclobutylmethyl]piperidine | Fluperlapine |
| 1-[2-(3,4-Dichlorophenyl)ethyl]-3-(pyrrolidin-1-yl)piperidine | Fluphenazine Decanoate DiHCl |
| 1-[2-(3,4-Dichlorophenyl)ethyl]-4-(3-phenylpropyl)piperazine | Fluphenazine Enanthate DiHCl |
| 1-[2-(3,4-Dichlorophenyl)ethyl]-4-methylpiperazine | Fluphenazine HCl |
| 1-[2-(4-Fluorophenyl)ethyl]-4,4-dimethylhexahydroazepine hydrochloride | Fluphenazine N-Mustard DiHCl |
| 1-[2-[1-(3,4-Dichlorophenyl)-5-methyl-1H-1,2,4-triazol-3-ylsulfanyl]ethyl]piperidine oxalate | Flurazepam Related Compound C |
| 1-[2-Benzyloxy-1 (R)-phenylethyl]-4-cyclohexylpiperazine dihydrochloride | Fluspirilene |
| 1-[3-(2-Oxo-3-phenylimidazolin-1-yl)propyl]spiro[piperidine-4, 1'(3H)-isobenzofuran] hydrochloride; 1-Phenyl-3-[3-[spiro[piperidine-4,1'(3H)-isobenzofuran]-1-yl]propyl]imidazolin-2-one hydrochloride | GBR 12783 DiHCl |
| 1-[3-(3,4-Dimethoxyphenyl)propyl]-4-(4-phenylbutyl)perhydro-1,4-diazepine dihydrochloride | GBR 12909 DiHCl |
| 1-[3-(4-Chlorophenoxy)propyl]-4-methylpiperidine hydrochloride | GBR 13069 DiHCl |
| 1-[3-(4-Phenyl-2H-1,2,3-triazol-2-yl)propyl]piperidine | GBR-12935 DiHCl |
| 1-[4-(6-Methoxynaphthalen-1-yl)butyl]-3,3-dimethylpiperidine hydrochloride | GR 89696 Fumarate |
| 1-[4-[2-[1-(3,4-Dichlorophenyl)-1H-pyrazol-3-yloxy]ethyl]piperazin-1-yl]ethanone oxalate | Guanabenz Acetate |
| 11-[5-(4-Fluorophenyl)-5-oxopentyl]-5,6,7,8,9,10-hexahydro-7,10-iminocyclohept[b]indole | Guanadrel Sulfate |
| 1-Benzyl-3beta-[3-(cyclopropylmethoxy)propyl]-2alpha,3alpha,4beta-trimethylpiperidine | Halofantrine HCl |
| 1-Benzyl-3-methoxy-3',4'-dihydrospiro(piperidine-4,1'-thieno[3,2-c]pyrane) | HEAT HCl |
| 1'-Benzyl-3-methoxy-4-phenyl-3,4-dihydrospiro[furo[3,4-c]pyrazole-1,4'-piperidine] | Hexylcaine HCl |
| 1-Benzyl-4-(4-fluorophenoxymethyl)piperidine | Hycanthone |
| 1-Benzyl-4-[2-(4-fluorophenyl)-2-oxoethyl]piperidine maleate | Hydroxychloroquine Sulfate |
| 1-Benzyl-4-[3-phenyl-2(E)-propenyloxymethyl]piperidine hydrochloride | IBZM, S(-)- |
| 1-Benzyl-4-[4-(4-fluorophenyl)-3-cyclohexen-1-yl]piperazine dihydrochloride hemihydrate | ICI-199,441 HCl |
| 1'-Benzylspiro[1,2,3,4-tetrahydronaphthalene-1,4'-piperidine] | Ifenprodil Tartrate |
| 1'-Benzylspiro[indane-1,4'-piperidine] | Indatraline HCl |
| 1 '-Butyl-3-Methoxy-4-phenyl-3,4-dihydrospiro[furo[3,4-c]pyrazole-1,4'-piperidine] | lofetamine HCl |
| 1-Cyclohexyl-4-(3-phenoxypropyl)piperazine dihydrochloride | Isamoltane Hemifumarate |
| 1-Hydroxy-1'-(2-phenylethyl)spiro[1,2,3,4-tetrahydronaphthalene-2,4'-piperidine] hydrochloride | Isoxsuprine HCl |
| 1-Methyl-4-[2-(4-phenylpiperidin-1-yl)ethyl]-4,5,6,7-tetrahydro-1H-indazole oxalate | Ketotifen Fumarate Salt |
| 1-Phenyl-3-(1-propyl-1,2,5,6-tetrahydropyridin-3-yl)-1-propanone oxime oxalate | L-693,403 Maleate |
| 1-Phenyl-4-(pyrrolidin-1-ylmethyl)-1,4,6,7-tetrahydropyrano[4,3-c]pyrazole | L-741,626 |
| 2-(2-{[1-(3-Chloro-Benzyl)-Pyrrolidin-3-Yl]-Methyl-Carbamoyl}-2-Methyl-Propyl)-4,6-Dimethyl-Benzoic Acid | L-741,742 HCl |
| 2-(3,4-Dichlorophenyl)-N-methyl-N-[2-(1,2alpha,3alpha,4beta-tetramethylpiperidin-3beta-yl)ethyl]acetamide | L-745,870 TriHCl |
| 2-(Cyclohexylmethylaminomethyl)-8-methoxy-3,4-dihydro-2H-1-benzopyran hydrochloride | Levetimide HCl, R(-) |
| 2(S)-[(3aS,6aR)-5-Butyl-4-oxo-1,2,3,3a,4,6a-hexahydrocyclopenta[c]pyrrol-2-yl]propionic acid ethyl ester | Levobunolol HCl |
| 2-[2-[5-Methyl-1-(2-naphthyl)-1 H-pyrazol-3-yloxy]ethylamino]ethanol hydrochloride | Lidoflazine |
| 2-[2-[N-(Cyclobutylmethyl)-N-methylamino]ethyl]-1,2,3,4-tetrahydronaphthalen-2-one | Lobeline HCl |
| 2-[3-[4-(2-Methoxyphenyl)piperazin-1-yl]propoxy]-9H-carbazole | Iomerizine diHCl |
| 2-[4-(4-Methoxybenzyl)piperazin-1-ylmethyl]-4H-1-benzopyran-4-one | Loxapine Succinate |
| 2-[N-[2-(3,4-Dichlorophenyl)ethyl]-N-methylaminomethyl]-1-ethylpyrrolidine | LY-53,857 Maleate |
| 2-Benzyl-3,4,8-trimethyl-2-azabicyclo[2.2.2]octane-6-carboxylic acid ethyl ester | Maprotiline HCl |
| 2-Butyl-2,3,4,4a,9,9a-hexahydro-1H-indeno[2,1-c]pyridine | Mazindol |
| 2-Chloro-11-(4-Methylpiperazino)Dibenz[B,F]Oxepin Maleate | MDL 12,330A HCl |
| 3-(1-Benzyl-2r,3c,4t-trimethylpiperidin-3t-yl)propionic acid ethyl ester hydrochloride | Mebhydroline 1,5-naphthalendisulfonate Salt |
| 3-(3-Chloro-4-cyclohexylphenyl)-1-(hexahydroazepin-1-yl)-1(Z)-propene hydrochloride; 1-[3-(3-Chloro-4-cyclohexylphenyl)-2(Z)-propenyl]hexahydroazepine hydrochloride | Meclizine HCl |
| 3-(4-Methylphenyl)-5-(1-propyl-1,2,5,6-tetrahydropyridin-3-yl)isoxazole oxalate | Mefloquine HCl |
| 3-(N-Benzyl-N-methylamino)-1-(4-nitrophenyl)piperidine | Meprylcaine HCl |
| 3,3'-Diethylthiacarbocyanine Iodide | Mesoridazine Besylate |
| 3-[1-(Benzocyclobutan-1-ylmethyl)piperidin-4-yl]-6-fluoro-1,2-benzisoxazole | Metaphit Methanesulfonate |
| 3-[2-(2-Adamantyl)ethyl]-3-azabicyclo[3.2.2]nonane | Metaphit |
| 3-[3-(4-Methylphenyl)isoxazol-5-yl]-1-propyl-1,2,5,6-tetrahydropyridine | Methantheline Bromide |
| 3a,6-Epoxy-2-[2-(4-fluorophenyl)ethyl]-2,3,3a,6,7,7a-hexahydro-1H-isoindole | Methdilazine |
| 3a,6-Epoxy-2-[2-(4-fluorophenyl)ethyl]perhydroisoindole | Methiothepin Mesylate |
| 3-Mercapto-2-Methylpropanoic Acid 1,2-Diphenylethylamine Salt | Methixene HCl |
| 3-Phenyl-1-(1-propyl-1,2,5,6-tetrahydro-3-pyridyl)-1-propanone oxime monohydrochloride | Methylene Violet 3Rax HCl |
| 3-Quinuclidinyl Benzilate | Metipranolol |
| 3-Tropanyl-3,5-Dichlorobenzoate | Mianserin HCl |
| 3-Tropanyl-Indole-3-Carboxylate HCl | Miconazole |
| 4-(1H-Indol-4-Yl)-Piperazine-1-Carboxylic Acid 2-(5-Bromo-2-Ethoxy-Phenylamino)-Cyclohexylmethyl Ester | ML-9 HCl |
| 4-(2-Tert-Butylsulfanyl-Benzyl)-Piperazine-1-Carboxylic Acid 2-Thiophen-2-Yl-Ethyl Ester | Morantel Hydrogen L-Tartrate |
| 4-(3,5-Dimethoxy-Phenyl)-Piperazine-1-Carboxylic Acid 1-(2-Fluoro-Benzyl)-Piperidin-2-Ylmethyl Ester | MR 16728 HCl |
| 4-(3-Nitro-5-Sulfamoyl-Thiophen-2-Yl)-Piperazine-1-Carboxylic Acid 1-(2-Fluoro-5-Methoxy-Benzyl)-Piperidin-3-Ylmethyl Ester | MT-210 |
| 4-(4-Benzylpiperazin-1-ylmethyl)-7-methoxy-2H-1-benzopyran-2-one | N-(2-Adamantyl)-N-[2-(2-adamantyl)ethyl]-N-methylamine hydrochloride |
| 4-(4-Bromophenyl)-5-[2-(dihexylamino)ethyl]thiazol-2-amine dihydrochloride | N-[1-(2-Indanyl)piperidin-4-yl]-N-methylcarbamic acid isobutyl ester fumarate |
| 4-(4-Fluorobenzoyl)-1-(4-Phenylbutyl)Piperidine Oxalate | N-[1-[4-Methoxy-3-(2-phenylethoxy)benzyl]-4-methylpentyl]-N-propylamine |
| 4-(4-Methylphenyl)-1-(3-morpholinopropyl)-1,2,3,6-tetrahydropyridine | N-[2-(3,4-Dichlorophenyl)ethyl]-N-ethyl-N-[2-(1-pyrrolidinyl)ethyl]amine |
| 4-(5-Trifluoromethyl-Pyridin-2-Yl)-Piperazine-1-Carboxylic Acid Pent-2-Ynyl Ester | N-[2-(3,4-Dichlorophenyl)ethyl]-N-methyl-N-(2-pyrrolidinoethyl)amine dihydrobromide |
| 4-(Dimethylamino)-1-phenylcyclohexanol | N-[4-[4-(Diethylamino)piperidin-1-yl]phenyl]methanesulfonamide |
| 4,7-Epoxy-2-[2-(4-fluorophenyl)ethyl]-2,3,3a,4,7,7a-hexahydro-1H-isoindole | N1-(1-Adamantyl)-N2-(2-methylphenyl)acetamidine |
| 4-[1-(3-[18F]fluoropropyl)piperidin-4-ylmethoxy]benzonitrile | N1-[2-(3,4-Dichlorophenyl)ethyl]-N1,N2,N2-trimethyl-1,2-ethanediamine |
| 4-[1-(4-Chlorobenzyl)-4-(benzylpiperidin-4-yl]-2-hydroxy-4-oxobut-2-enoic acid | Nafronyl Oxalate Salt |
| 4-[1-(4-Fluorophenyl)-1-hydroxymethyl]-1-[3-(4-fluorophenoxy)propyl]piperidine | Naftifine |
| 4-[2-(Dipropylamino)ethyl]-2-(2-phenylethoxy)anisole hydrochloride | Naftopidil diHCl |
| 4-[2-(Dipropylamino)ethyl]-5,8-dimethylcarbazole hydrochloride | Naltriben Mesylate |
| 4-[2-[1-(3,4-Dichlorophenyl)-5-methyl-1H-pyrazol-3-yloxy]ethyl]morpholine | NE-100 |
| 4-[2-[1-(Cyclopropylmethyl)piperidin-4-yl]acetyl]benzonitrile fumarate | Nefazodone |
| 4-[4-(N-Benzyl-N-methylamino)piperidin-1-yl]benzonitrile | N-Ethyl-N-[2-(1-piperidinyl)ethyl]-N-[2-[4-(trifluoromethoxy)phenyl]ethyl]amine |
| 4-[N-[2-[N'-(4-Fluorobenzyl)-N'-methylamino]ethyl]-N-methylamino]-1-(4-fluorophenyl)-1-butanone dihydrochloride | Nicergoline |
| 4-Benzyl-1-[4-(4-fluorophenyl)-4-hydroxybutyl]piperidine hydrochloride | Niguldipine HCl, (+/-)- |
| 4-Bromo-N-[1-(9-Ethyl-9H-Carbazol-3-Ylmethyl)-Pyrrolidin-3-Yl]-2-Trifluoromethoxy-Benzenesulfonamide | Nisoxetine HCl |
| 4'-Chloro-3-Alpha-(Diphenylmethoxy)Tropane HCl | NP-07 |
| 4-Furan-2-Ylmethyl-Piperazine-1-Carboxylic Acid 2-{4-[3-(2-Trifluoromethyl-Phenothiazin-10-Yl)-Propyl]-Piperazin-1-Yl}-Ethyl Ester | Nylidrin HCl |
| 4-Methoxy-1-[2-(4-phenylpiperazin-1-yl)ethyl]-6H-dibenzo[b,d]pyran hydrochloride | Octoclothepin Maleate, (±)- |
| 4-Methoxy-N-[1-(7-Methoxy-Benzo[1,3]Dioxol-5-Ylmethyl)-Pyrrolidin-3-Yl]-Benzenesulfonamide | Oxamniquine |
| 4-Phenyl-1-(3-phenylpropyl)-4-(pyrrolidin-1-ylcarbonyl)piperidine | Oxamniquine Related Compound A |
| 5-(2-Pyrrolidinoethyl)-4-(2,4,6-trimethoxyphenyl)thiazole-2-amine dihydrochloride | Oxamniquine Related Compound B |
| 5-(N-Ethyl-N-Isopropyl)-Amiloride | Oxatomide |
| 6-[1-Hydroxy-2-[4-(2-phenylethyl)piperidin-1-yl]ethyl]-1,2,3,4-tetrahydroquinolin-2-one | Oxiconazole Nitrate |
| 6-[2-(4-Benzylpiperidin-1-yl)ethyl]-3-methylbenzothiazol-2(3H)-one | Panamesine hydrochloride |
| 6-[2-[4-(2-Phenylethyl)piperidin-1-yl]ethyl]-1,2,3,4-tetrahydroquinolin-2-one | Panaxatriol |
| 6-[3-(Morpholin-4-yl)propyl]benzothiazol-2(3H)-one | PAPP |
| 6-[6-(4-Hydroxypiperidin-1-yl)hexyloxy]-3-methyl-2-phenyl-4H-1-benzopyran-4-one | Paroxetine |
| 7-(4-Methoxyphenyl)-4-[4-(4-pyridyl)butyl]hexahydro-1,4-thiazepine | Paxilline |
| 7-[3-[4-(4-Fluorobenzoyl)piperidin-1-yl]propoxy]-4H-1-benzopyran-4-one hydrochloride | p-Chlorobenzhydrylpiperazine |
| 9-[4-({[4'-(trifluoromethyl)-1,1'-biphenyl-2-yl]carbonyl}amino)piperidin-1-yl]-N-(2,2,2-trifluoroethyl)-9H-fluorene-9-carboxamide | Penbutolol Sulfate |
| 9-Hydroxy-2,3,6,7,7a,8,12b,12c-octahydro-1H,5H-naphtho[1,2,3-ij]quinolizine | Pentamidine Isethionate |
| Acetophenazine Maleate | Pergolide Methanesulfonate |
| Acrinol | Perospirone |
| Ajmaline | Phenamil Methanesulfonate |
| Alaproclate HCl | Phenosafranin HCl |
| Aloe-Emodin | Piboserod |
| Alprenolol D-Tartrate Salt Hydrate | Pimozide |
| Alprenolol HCl | Pinacyanol Chloride |
| AMI-193 | Pindobind, (+/-)- |
| Aminobenztropine | Piperacetazine |
| Amiodarone HCl | Piperidolate HCl |
| Amodiaquine HCl | Pirenperone |
| Amorolfine HCl | PPHT HCl, (±)- |
| Amoxapine | Prenylamine Lactate Salt |
| AN2/AVex-73; AE-37; ANAVEX 2-73; N-(2,2-Diphenyltetrahydrofuran-3-ylmethyl)-N,N-dimethylamine | Pridinol Methanesulfonate Salt |
| Anavex 1-41; AE-14; N-(5,5-Diphenyltetrahydrofuran-3-ylmethyl)-N,N-dimethylamine hydrochloride | Procyclidine HCl |
| Anavex 19-144; AE-37met; AN19/AVex-144 | Proflavine Hemisulfate Salt |
| Anavex 7-1037 | Propafenone HCl |
| Anisotropine Methylbromide | Proparacaine HCl |
| Anpirtoline | Propiomazine |
| ARC 239 DiHCl | Protokylol |
| Auramine O HCl | Protriptyline HCl |
| Azaperone | Pyrilamine Maleate |
| Azatadine Maleate | Pyrimethamine |
| Azelastine HCl | Pyrrolidine-1,2-Dicarboxylic Acid 1-[1-(4-Allyloxy-Benzyl)-Piperidin-2-Ylmethyl] Ester 2-Benzyl Ester |
| Bamethan sulfate | Pyrvinium Pamoate |
| BD 1008 DiHBr | Quetiapine Fumarate |
| BD-1063 | Quinacrine HCl |
| Benextramine TetraHCl | Quinaldine Red |
| Benfluorex HCl | Quipazine Dimaleate |
| Benidipine HCl | Quipazine, 6-Nitro-, Maleate |
| Benoxathian HCl | Raloxifene |
| Benproperine Phosphate | Rimantadine HCl |
| Benzododecinium bromide | Rimcazole hydrochloride |
| Benzphetamine HCl | Risperidone |
| Benztropine Mesylate | Ritanserin |
| Bephenium Hydroxynaphthoate | Ritodrine HCl |
| Bepridil HCl | RS 23597-190 HCl |
| Berberine chloride | RS 67333 HCl |
| Betaxolol HCl | RS 67506 HCl |
| Bifemelane | Safranin O HCl |
| BMY 7378 DiHCl | Salmeterol |
| Bopindolol Malonate | SB203186 |
| BP 554 Maleate | SCH-23390 HCl, R(+)- |
| Bromhexine HCl | Sertaconazole Nitrate |
| Bromodiphenhydramine HCl | Sertindole |
| Bromperidol | Sertraline |
| Brompheniramine Maleate | Sibutramine HCl |
| BTCP HCl | Siramesine hydrochloride |
| Buclizine HCl | SKF-525A HCl |
| Buflomedil HCl | SKF-96365 HCl |
| Bupropion HCl | SNC 121 |
| Buspirone HCl | Spiperone HCl |
| Butacaine Sulfate | T-226296 |
| Butaclamol HCl, (±)- | Tegaserod Maleate |
| Butenafine HCl | Terbinafine HCl |
| Butoconazole Nitrate | Terconazole |
| BW 723C86 HCl | Terfenadine |
| Carbetapentane Citrate | Terfenadine Related Compound A |
| Carbinoxamine Maleate | Tetrindole Mesylate |
| Carpipramine DiHCl DiH2O | Thiethylperazine Malate |
| Carvedilol | Thioperamide Maleate |
| Cephapirin Benzathine | Thioproperazine |
| CGS-12066A Maleate | Thioridazine |
| Chloroprocaine HCl | Thiothixene |
| Chlorpheniramine Maleate | Thiothixene, (E)- |
| Chlorphenoxamine HCl | Thonzonium Bromide |
| Chlorprothixene | Tioconazole Related Compound A |
| Cinanserin HCl | TMB-8 HCl |
| Cinnarizine | Tolterodine L-Tartrate |
| Cirazoline HCl | Toremifene Citrate |
| Cis-(+/-)-N-Methyl-N-[2-(3,4-Dichlorophenyl)Ethyl]-2-(1-Pyrrolidinyl)Cyclohexamine DiHBr | Tramazoline HCl |
| Cis(Z)-Flupentixol DiHCl | Trans-U-50488 Methanesulfonate, (±)- |
| cis-2-(Cyclopropylmethyl)-7-(4-fl uorobenzoyl)perhydropyrido[1 ,2-a] pyrazi ne | Tridihexethyl Chloride |
| cis-2-[4-(Trifluoromethyl)benzyl]-3a,4,7,7a-tetrahydroisoindoline | Trifluoperazine HCl |
| Cisapride Hydrate | Trifluperidol HCl |
| Citalopram HBr | Trihexyphenidyl HCl |
| Clemastine Fumarate | Trimeprazine Hemi-L-Tartrate |
| Clemizole HCl | Trimipramine Maleate |
| Clenbuterol HCl | Tripelennamine HCl |
| Clidinium Bromide | Triprolidine HCl |
| Clobenpropit 2HBr | Triprolidine HCl Z Isomer |
| Clofazimine | Tropanyl 3,5-Dimethylbenzoate |
| Clofilium Tosylate | Tropine 2-(4-Chlorophenoxy)Butanoate, Maleate |
| Clomiphene Citrate | U-50488 HCl, (-)- |
| Clomiphene Related Compound A | U-62066 |
| Clomipramine | UH 232 Maleate, (+)- |
| Cloperastine HCl | Vesamicol HCl |
| Clorgyline HCl | Vinpocetine |
| Clozapine | W-7 HCl |
| Conessine | WB-4101 HCl |

Preferably, the table above includes also reduced haloperidol. Reduced haloperidol is an active metabolite of haloperidol that is produced in humans, shows a high affinity (in the low nanomolar range) for sigma-1 receptors, and produces an irreversible blockade of sigma-1 receptors both in experimental animals and human cells.

Examples of well known methods of producing a prodrug of a given acting compound are known to those skilled in the art (e.g. in Krogsgaard-Larsen et al., Textbook of Drug design and Discovery, Taylor & Francis (April 2002)).

In a preferred embodiment, the sigma ligand in the context of the present invention has the general formula (I) as depicted above.

In a preferred embodiment, R₁ in the compounds of formula (I) is selected from H, - COR₈, and substituted or unsubstituted alkyl. More preferably, R₁ is selected from H, methyl and acetyl. A more preferred embodiment is when R₁ is H.

In another preferred embodiment, R₂ in the compounds of formula (I) represents H or alkyl, more preferably methyl.

In yet another preferred embodiment of the invention, R₃ and R₄ in the compounds of formula (I) are situated in the meta and para positions of the phenyl group, and preferably, they are selected independently from halogen and substituted or unsubstituted alkyl.

In an especially preferred embodiment of the invention, in the compounds of formula (I) both R₃ and R₄ together with the phenyl group form an optionally substituted fused ring system (for example, a substituted or unsubstituted aryl group or a substituted or unsubsituted, aromatic or non-aromatic heterocyclyl group may be fused), more preferably, a naphthyl ring system.

Also in the compounds of formula (I), embodiments where n is selected from 2, 3, 4 are preferred in the context of the present invention, more preferably n is 2.

Finally, in another embodiment it is preferred in the compounds of formula (I) that R₅ and R₆ are, each independently, C₁₋₆ alkyl, or together with the nitrogen atom to which they are attached form a substituted or unsubstituted heterocyclyl group a, in particular a group chosen among morpholinyl, piperidinyl, and pyrrolidinyl group. More preferably, R₅ and R₆ together form a morpholine-4-yl group.

In preferred variants of the invention, the sigma ligand of formula (I) is selected from:
[1] 4-{2-(1-(3,4-dichiorophenyl)-5-methyl-1H pyrazol-3-yloxy)ethyl}morpholine
[2] 2-[1-(3,4-Dichlorophenyl)-5-methyl-1H-pyrazol-3-yloxy]-N,N-diethylethanamine
[3] 1-(3,4-Dichlorophenyl)-5-methyl-3-[2-(pyrrolidin-1-yl)ethoxy]-1H-pyrazole
[4] 1-(3,4-Dichlorophenyl)-5-methyl-3-[3-(pyrrolidin-1-yl)propoxy]-1H-pyrazole
[5] 1-{2-[1-(3,4-Dichlorophenyl)-5-methyl-1H-pyrazol-3-yloxy]ethyl}piperidine
[6] 1-{2-[1-(3,4-dichlorophenyl)-5-methyl-1H-pyrazol-3-yloxy]ethyl}-1H-imidazole
[7] 3-{1-[2-(1-(3,4-Dichlorophenyl)-5-methyl-1H-pyrazol-3-yloxy)ethyl]piperidin-4-yl}-3H-imidazo[4,5-b]pyridine
[8] 1-{2-[1-(3,4-Dichlorophenyl)-5-methyl-1H-pyrazol-3-yloxy]ethyl}-4-methylpiperazine
[9] Ethyl 4-{2-[1-(3,4-dichlorophenyl)-5-methyl-1H-pyrazol-3-yloxy]ethyl}piperazine carboxylate
[10] 1-(4-(2-(1-(3,4-dichlorophenyl)-5-methyl-1H-pyrazol-3-yloxy)ethyl)piperazin-1-yl)ethanone
[11] 4-{2-[1-(4-Methoxyphenyl)-5-methyl-1H-pyrazol-3-yloxy]ethyl}morpholine
[12] 1-(4-Methoxyphenyl)-5-methyl-3-[2-(pyrrolidin-1-yl)ethoxy]-1H-pyrazole
[13] 1-(4-Methoxyphenyl)-5-methyl-3-[3-(pyrrolidin-1-yl)propoxy]-1H-pyrazole
[14] 1-[2-(1-(4-Methoxyphenyl)-5-methyl-1H-pyrazol-3-yloxy)ethyl]piperidine
[15] 1-{2-[1-(4-Methoxyphenyl)-5-methyl-1H-pyrazol-3-yloxy]ethyl}-1H-imidazole
[16] 4-{2-[1-(3,4-Dichlorophenyl)-5-phenyl-1H-pyrazol-3-yloxy]ethyl}morpholine
[17] 1-(3,4-Dichlorophenyl)-5-phenyl-3-[2-(pyrrolidin-1-yl)ethoxy]-1H-pyrazole
[18] 1-(3,4-Dichlorophenyl)-5-phenyl-3-[3-(pyrrolidin-1-yl)propoxy]-1H-pyrazole
[19] 1-{2-[1-(3,4-Dichlorophenyl)-5-phenyl-1H-pyrazol-3-yloxy]ethyl}piperidine
[20] 1-{2-[1-(3,4-Dichlorophenyl)-5-phenyl-1H-pyrazol-3-yloxy]ethyl}-1H-imidazole
[21]2-{2-[1-(3,4-dichlorophenyl)-5-phenyl-1H-pyrazol-3-yloxy]ethyl}-1,2,3,4-tetrahydroisoquinoline
[22] 4-{4-[1-(3,4-Dichlorophenyl)-5-methyl-1H-pyrazol-3-yloxy]butyl}morpholine
[23] 1-(3,4-Dichlorophenyl)-5-methyl-3-[4-(pyrrolidin-1-yl)butoxy]-1H-pyrazole
[24] 1-{4-[1-(3,4-Dichlorophenyl)-5-methyl-1H-pyrazol-3-yloxy]butyl}piperidine
[25]1-{4-[1-(3,4-Dichlorophenyl)-5-methyl-1H-pyrazol-3-yloxy]butyl}-4-methylpiperazine
[26] 1-{4-[1-(3,4-Dichlorophenyl)-5-methyl-1H-pyrazol-3-yloxy]butyl}-1H-imidazole
[27] 4-[1-(3,4-Dichlorophenyl)-5-methyl-1H-pyrazol-3-yloxy]-N,N-diethylbutan-1-amine
[28]1-{4-[1-(3,4-dichlorophenyl)-5-methyl-1H-pyrazol-3-yloxy]butyl}-4-phenylpiperidine
[29] 1-{4-[1-(3,4-dichlorophenyl)-5-methyl-1H-pyrazol-3-yloxy]butyl}-6,7-dihydro-1H-indol-4(5H)-one
[30] 2-{4-[1-(3,4-dichlorophenyl)-5-methyl-1H-pyrazol-3-yloxy]butyl}-1,2,3,4-tetrahydroisoquinoline
[31] 4-{2-[1-(3,4-dichlorophenyl)-5-isopropyl-1H-pyrazol-3-yloxy]ethyl}morpholine
[32]2-[1-(3,4-Dichlorophenyl)-5-isopropyl-1H-pyrazol-3-yloxy]-N,N-diethylethanamine
[33] 1-(3,4-Dichlorophenyl)-5-isopropyl-3-[2-(pyrrolidin-1-yl)ethoxy]-1H-pyrazole
[34] 1-(3,4-Dichlorophenyl)-5-isopropyl-3-[3-(pyrrolidin-1-yl)propoxy]-1H-pyrazole
[35] 1-{2-[1-(3,4-Dichiorophenyl)-5-isopropyl-1H-pyrazol-3-yloxy]ethyl}piperidine
[36] 2-{2-[1-(3,4-dichlorophenyl)-5-isopropyl-1H-pyrazol-3-yloxy]ethyl}-1,2,3,4-tetrahydroisoquinoline
[37] 4-{2-[1-(3,4-dichlorophenyl)-1H-pyrazol-3-yloxy]ethyl}morpholine
[38] 2-[1-(3,4-dichlorophenyl)-1H-pyrazol-3-yloxy] N,N-diethylethanamine
[39] 1-(3,4-dichlorophenyl)-3-[2-(pyrrolidin-1-yl)ethoxy]-1H-pyrazole
[40] 1-{2-[1-(3,4-dichlorophenyl)-1H-pyrazol-3-yloxy]ethyl}piperidine
[41] 1-(3,4-dichlorophenyl)-3-[3-(pyrrolidin-1-yl)propoxy]-1H-pyrazole
[42]1-{2-[1-(3,4-Dichlorophenyl)-5-methyl-1H-pyrazol-3-yloxy]ethyl}piperazine
[43] 1-{2-[1-(3,4-Dichlorophenyl)-5-methyl-1H-pyrazol-3-yloxy]ethyl}pyrrolidin-3-amine
[44]4-{2-[1-(3,4-Dichlorophenyl)-4,5-dimethyl-1H-pyrazol-3-yloxy]ethyl}morpholine
[46]2-[1-(3,4-Dichlorophenyl)-4,5-dimethyl-1H-pyrazol-3-yloxy]-N,N-diethylethanamine
[47] 1-(3,4-Dichlorophenyl)-4,5-dimethyl-3-[2-(pyrrolidin-1-yl)ethoxy]-1H-pyrazole
[48] 1-(3,4-Dichlorophenyl)-4,5-dimethyl-3-[3-(pyrrolidin-1-yl)propoxy]-1H-pyrazole
[49] 1-{2-[1-(3,4-Dichlorophenyl)-4,5-dimethyl-1H-pyrazol-3-yloxy]ethyl}piperidine
[50] 4-{4-[1-(3,4-dichlorophenyl)-1H-pyrazol-3-yloxy]butyl}morpholine
[51](2S,6R)-4-{4-[1-(3,4-dichlorophenyl)-1H-pyrazol-3-yloxy]butyl}-2,6-dimethylmorpholine
[52] 1-{4-[1-(3,4-Dichlorophenyl)-1H-pyrazol-3-yloxy]butyl}piperidine
[53] 1-(3,4-Dichlorophenyl)-3-[4-(pyrrolidin-1-yl)butoxy]-1H-pyrazole
[55] 4-[1-(3,4-dichlorophenyl)-1H-pyrazol-3-yloxy]-N,N-diethylbutan-1-amine
[56] N-benzyl-4-[1-(3,4-dichlorophenyl)-1H-pyrazol-3-yloxy]-N-methylbutan-1-amine
[57]4-[1-(3,4-dichlorophenyl)-1H-pyrazol-3-yloxy]-N-(2-methoxyethyl)-N-methylbutan-1-amine
[58] 4-{4-[1-(3,4-dichlorophenyl)-1H-pyrazol-3-yloxy]butyl}thiomorpholine
[59]1-[1-(3,4-Dichlorophenyl)-5-methyl-3-(2-morpholinoethoxy)-1H-pyrazol-4-yl]ethanone
[60]1-{1-(3,4-dichlorophenyl)-5-methyl-3-[2-(pyrrolidin-1-yl)ethoxy]-1H-pyrazol-4-yl}ethanone
[61] 1-{1-(3,4-dichlorophenyl)-5-methyl-3-[2-(piperidin-1-yl)ethoxy]-1H-pyrazol-4-yl}ethanone
[62] 1-{1-(3,4-dichlorophenyl)-3-[2-(diethylamino)ethoxy]-5-methyl-1H-pyrazol-4-yl}ethanone
[63] 4-{2-[5-Methyl-1-(naphthalen-2-yl)-1H-pyrazol-3-yloxy]ethyl}morpholine
[64] N,N-Diethyl-2-[5-methyl-1-(naphthalen-2-yl)-1H-pyrazol-3-yloxy]ethanamine
[65] 1-{2-[5-Methyl-1-(naphthalen-2-yl)-1H-pyrazol-3-yloxy]ethyl}piperidine
[66] 5-Methyl-1-(naphthalen-2-yl)-3-[2-(pyrrolidin-1-yl)ethoxy]-1H-pyrazole
   or their pharmaceutically acceptable salts, isomers, prodrugs or solvates.

In a preferred embodiment of the invention, the sigma ligand of formula (I) is 4-{2-[5-Methyl-1-(naphthalen-2-yl)-1H-pyrazol-3-yloxy]ethyl} morpholine. This particular compound is designated in the examples of the present invention as compound n° 63.

In a more preferred embodiment, the sigma ligand of formula (I) is the 4-{2-[5-Methyl-1-(naphthalen-2-yl)-1H-pyrazol-3-yloxy]ethyl} morpholine hydrochloride. This particular compound is designated in the examples of the present invention as example n° 1.

The compounds of formula (I) and their salts or solvates can be prepared as disclosed in the previous application WO2006/021462.

As stated previously, one aspect of this invention refers to the use of sigma ligand as defined above for the manufacture of a medicament for the prevention and/or treatment of pain associated to bone cancer (i.e. bone cancer pain). Preferably, the pain is selected from acute and/or chronic pain developed as a consequence of bone cancer, preferably neuropathic pain, neuralgia, allodynia, causalgia, hyperalgesia, hyperesthesia, hyperpathia, neuritis or neuropathy secondary to surgical procedure.

It has found also that there is a significant synergistic effect when a sigma ligand and an opioid or opiate compound are combined: Sub-threshold dose of morphine was given with example 1 treatment and resulted in an increase in analgesia 30 minutes after administration and the effect lasted at least 24 hours (the positive control, 5mg/kg of morphine, showed no effect 24 hours after administration). Thus, the invention is also directed to a combination of at least one sigma ligand as defined above and at least one opioid or opiate compound for simultaneous, separate or sequential administration, for use in the prevention and/or treatment of pain associated to bone cancer. Compounds that bind to the opioid receptor within the scope of the present invention include natural opiates, such as morphine, codeine and thebaine; semisynthetic opiates, derived from the natural opioids, such as hydromorphone, hydrocodone, oxycodone, oxymorphone, desomorphine, diacetylmorphine, nicomorphine, dipropanoylmorphine, benzylmorphine and ethylmorphine; fully synthetic opioids, such as fentanyl, pethidine, methadone, tramadol and propoxyphene; and endogenous opioid peptides, produced naturally in the body, such as endorphins, enkephalins, dynorphins, and endomorphins and their analogues. Preferably, the combination according to this invention comprises morphine or its analogues.

The combination of the invention may be formulated for its simultaneous separate or sequential administration, with at least a pharmaceutically acceptable carrier, additive, adjuvant or vehicle. This has the implication that the combination of the two active compounds may be administered:
a) As a combination that is being part of the same medicament formulation, the two active compounds being then administered always simultaneously.
b) As a combination of two units, each with one of the active substances giving rise to the possibility of simultaneous, sequential or separate administration. In a particular embodiment, the sigma ligand is independently administered from the opioid or opiate (i.e in two units) but at the same time. In another particular embodiment, the sigma ligand is administered first, and then the opioid or opiate is separately or sequentially administered. In yet another particular embodiment, the opioid or opiate is administered first, and then the sigma ligand is administered, separately or sequentially, as defined.

The auxiliary materials or additives of a pharmaceutical composition according to the present invention (i.e. a composition comprising at least one sigma ligand or a composition comprising at least one sigma ligand and at least one opioid or opiate compound) can be selected among carriers, excipients, support materials, lubricants, fillers, solvents, diluents, colorants, flavour conditioners such as sugars, antioxidants, binders, adhesives, disintegrants, anti-adherents, glidants and/or agglutinants. In the case of suppositories, this may imply waxes or fatty acid esters or preservatives, emulsifiers and/or carriers for parenteral application. The selection of these auxiliary materials and/or additives and the amounts to be used will depend on the form of application of the pharmaceutical composition.

The pharmaceutical composition in accordance with the invention can be adapted to any form of administration, be it orally or parenterally, for example pulmonar, nasally, rectally and/or intravenously. Therefore, the formulation in accordance with the invention may be adapted for topical or systemic application, particularly for dermal, transdermal, subcutaneous, intramuscular, intra-articular, intraperitoneal, intravenous, intra-arterial, intravesical, intraosseous, intracavernosal, pulmonary, buccal, sublingual, ocular, intravitreal, intranasal, percutaneous, rectal, vaginal, oral, epidural, intrathecal, intraventricular, intracerebral, intracerebroventricular, intracisternal, intraspinal, perispinal, intracranial, delivery via needles or catheters with or without pump devices, or other application routes.

Suitable preparations for oral applications are tablets, pills, caplets, gel caps, chewing gums, capsules, granules, drops or syrups.

Suitable preparations for parenteral applications are solutions, suspensions, reconstitutable dry preparations, aerosols or sprays.

The composition of the invention may be formulated as deposits in dissolved form or in patches, for percutaneous application.

Skin applications include ointments, gels, creams, lotions, suspensions or emulsions.

Suitable form of rectal application is by means of suppositories.

Moreover, the composition may be presented in a form suitable for once daily, weekly, or monthly administration.

Accordingly, in another aspect the invention provides a method of treatment of a patient, notably a human, suffering from pain associated to bonce cancer, or likely to suffer pain as a result of bone cancer, which comprises administering to the patient in need of such a treatment or prophylaxis a therapeutically effective amount of a sigma ligand as defined above.

In one embodiment of the invention it is preferred that the sigma ligand is used in therapeutically effective amounts. The physician will determine the dosage of the present therapeutic agents which will be most suitable and it will vary with the form of administration and the particular compound chosen, and furthermore, it will vary with the patient under treatment, the age of the patient, the type of condition being treated. He will generally wish to initiate treatment with small dosages substantially less than the optimum dose of the compound and increase the dosage by small increments until the optimum effect under the circumstances is reached. When the composition is administered orally, larger quantities of the active agent will be required to produce the same effect as a smaller quantity given parenterally. The compounds are useful in the same manner as comparable therapeutic agents and the dosage level is of the same order of magnitude as is generally employed with these other therapeutic agents.

For example, the dosage regime that must be administered to the patient will depend on the patient's weight, the type of application, the condition and severity of the disease. A preferred dosage regime of comprises an administration of a compound according the present invention within a range of 0.01 to 300 mg/kg, more preferably 0.01 to 100 mg/kg, and most preferable 0.01 to 50 mg/kg.

The following examples are merely illustrative of certain embodiments of the invention and cannot be considered as restricting it in any way.

### Examples

### Example 1. Synthesis of 4-{2-[5-Methyl-1-(naphthalen-2-yl)-1H-pyrazol-3-yloxy]ethyl} morpholine (compound 63) and its hydrochloride salt

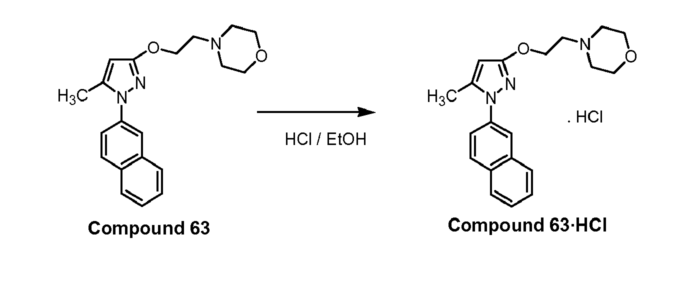

Compound 63 can be can be prepared as disclosed in the previous application WO2006/021462. Its hydrochloride can be obtained according the following procedure:

Compound 63 (6,39 g) was dissolved in ethanol saturated with HCl, the mixture was stirred then for some minutes and evaporated to dryness. The residue was crystallized from isopropanol. The mother liquors from the first crystallization afforded a second crystallization by concentrating. Both crystallizations taken together yielded 5.24 g (63 %) of the corresponding hydrochloride salt (m.p. = 197-199 °C).

¹H-NMR (DMSO-d₆) δ ppm: 10,85 (bs, 1H), 7,95 (m, 4H), 7,7 (dd, J=2,2, 8,8 Hz, 1H), 7,55 (m, 2H), 5,9 (s, 1H), 4,55 (m, 2H), 3,95 (m, 2H), 3,75 (m, 2H), 3,55-3,4 (m, 4H), 3,2 (m, 2H), 2,35 (s, 3H).

HPLC purity: 99.8%.

### Pharmacological data

In this assay, the use of the example 1 for treatment of tumor-induced bone pain was evaluated using a syngeneic rat model of cancer-induced bone pain. The rat mammary gland carcinoma cell line MRMT-1 was injected into the marrow space of the proximal tibia and the animals were treated on day 18 with vehicle, a reference compound (morphine), or example 1.

Example 1 efficacy was determined from using the von Frey test for the measurement of tactile allodynia at baseline, pre and post 18 day dosing for Phase 1 and baseline, pre, post 18 day and post 19 day (24 hour) dosing for Phase 2.

This study was conducted in two parts; the first phase determined the optimal dose level for the second phase. The second phase was to explore the potential synergistic effect of treatment with morphine and example 1.

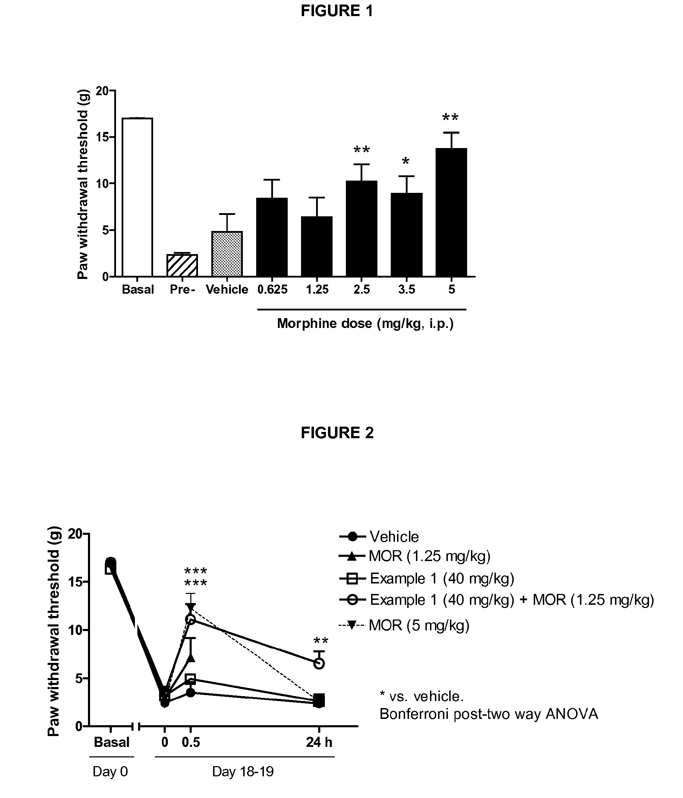

In the first phase of the study, various doses of morphine (Figure 1) were given at day 18 and only the dose of 2.5 and 5 mg/kg were able to mitigate allodynia with statistical significance. Based on these results, the sub threshold dose of 1.25 mg/kg was chosen for phase 2 of the study to look at potential synergy with example 1 treatment. Morphine at 5 mg/kg was chosen as positive control in the phase 2 study.

In the second phase of the study, example 1 alone was able to demonstrate statistically significant mitigation of allodynia 30 minutes after administration at 80 mg/kg (similar to 5mg/kg of morphine) but not at 40mg/kg. Sub-threshold dose of morphine (1.25 mg/kg) was given with example 1 treatment (80 and 40 mg/kg) and resulted in an increase in analgesia 30 minutes after administration and the effect lasted at least 24 hours. In contrast, 5 mg/kg of morphine showed no significant effect 24 hours after administration.

In summary, example 1 in combination with morphine produces a synergistic analgesic effect that lasts at least 24 hours in a bone cancer pain model.

## Claims

1. A sigma ligand for use in the prevention and/or treatment of bone cancer pain.

2. Sigma ligand according to claim 1, wherein the pain is selected from acute and/or chronic pain developed as a consequence of bone cancer, preferably neuropathic pain, neuralgia, allodynia, causalgia, hyperalgesia, hyperesthesia, hyperpathia, neuritis or neuropathy secondary to surgical procedure.

3. Sigma ligand according to claims 1 or 2, which is selected from a sigma receptor antagonist preferably selected from a neutral antagonist, an inverse agonist or a partial antagonist.

4. Sigma ligand according to any preceding claim, having the general formula (I): 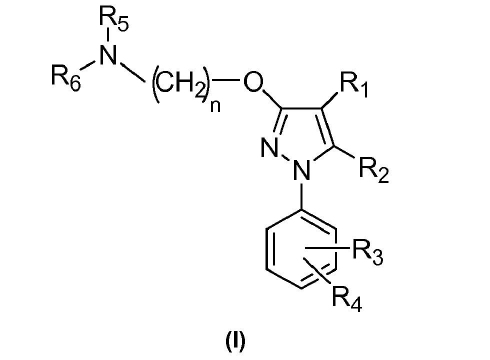 wherein
**R₁** is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted non-aromatic heterocyclyl, substituted or unsubstituted aromatic heterocyclyl, substituted or unsubstituted heterocyclylalkyl, -COR₈, -C(O)OR₈, - C(O)NR₈R₉, -CH=NR₈, -CN, -OR₈, -OC(O)R₈, -S(O)ₜ-R₈, -NR₈R₉, - NR₈C(O)R₉, -NO₂, -N=CR₈R₉, and halogen;
**R₂** is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, - COR₈, -C(O)OR₈, -C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉, and halogen;
**R₃** and **R₄** are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈, -C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉, and halogen, or together they form an optionally substituted fused ring system;
**R₅** and **R₆** are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈, -C(O)NR₈R₉, -CH=NR₈, -CN, -OR₈, -OC(O)R₈, -S(O)ₜ-R₈, -NR₈R₉, -NR₈C(O)R₉, -NO₂, -N=CR₈R₉, and halogen, or together form, with the nitrogen atom to which they are attached, a substituted or unsubstituted, aromatic or non-aromatic heterocyclyl group;
**n** is selected from 1, 2, 3, 4, 5, 6, 7 and 8;
**t** is 1,2 or 3;
**R₈** and **R₉** are each independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted, aromatic or non-aromatic heterocyclyl, substituted or unsubstituted alkoxy, substituted or unsubstituted aryloxy, and halogen;
or a pharmaceutically acceptable salt, isomer, prodrug or solvate thereof.

5. Sigma ligand according to claim 4, wherein R₁ is selected from H, -COR₈ and substituted or unsubstituted alkyl.

6. Sigma ligand according to claims 4 or 5, wherein R₂ is H or alkyl.

7. Sigma ligand according to any of claims 4 to 6, wherein R₃ and R₄ together form a fused naphthyl ring system.

8. Sigma ligand according to any of claims 4 to 7, wherein R₅ and R₆ together form a morpholine-4-yl group.

9. Sigma ligand according to any preceding claim, which is selected from the group consisting of:
4-{2-(1-(3,4-dichlorophenyl)-5-methyl-1H pyrazol-3-yloxy)ethyl}morpholine,
2-[1-(3,4-Dichlorophenyl)-5-methyl-1H-pyrazol-3-yloxy]-N,N-diethylethanamine,
1-(3,4-Dichlorophenyl)-5-methyl-3-[2-(pyrrolidin-1-yl)ethoxy]-1H-pyrazole,
1-(3,4-Dichlorophenyl)-5-methyl-3-[3-(pyrrolidin-1-yl)propoxy]-1H-pyrazole,
1-{2-[1-(3,4-Dichlorophenyl)-5-methyl-1H-pyrazol-3-yloxy]ethyl}piperidine,
1-{2-[1-(3,4-dichlorophenyl)-5-methyl-1H-pyrazol-3-yloxy]ethyl}-1H-imidazole,
3-{1-[2-(1-(3,4-Dichlorophenyl)-5-methyl-1H-pyrazol-3-yloxy)ethyl]piperidin-4-yl}-3H-imidazo[4,5-b]pyridine,
1-{2-[1-(3,4-Dichlorophenyl)-5-methyl-1H-pyrazol-3-yloxy]ethyl}-4-methylpiperazine,
Ethyl 4-{2-[1-(3,4-dichlorophenyl)-5-methyl-1H-pyrazol-3-yloxy]ethyl}piperazine carboxylate,
1-(4-(2-(1-(3,4-dichlorophenyl)-5-methyl-1H-pyrazol-3-yloxy)ethyl)piperazin-1-yl)ethanone,
4-{2-[1-(4-Methoxyphenyl)-5-methyl-1H-pyrazol-3-yloxy]ethyl}morpholine,
1-(4-Methoxyphenyl)-5-methyl-3-[2-(pyrrolidin-1-yl)ethoxy]-1H-pyrazole,
1-(4-Methoxyphenyl)-5-methyl-3-[3-(pyrrolidin-1-yl)propoxy]-1H-pyrazole,
1-[2-(1-(4-Methoxyphenyl)-5-methyl-1H-pyrazol-3-yloxy)ethyl]piperidine,
1-{2-[1-(4-Methoxyphenyl)-5-methyl-1H-pyrazol-3-yloxy]ethyl}-1H-imidazole,
4-{2-[1-(3,4-Dichlorophenyl)-5-phenyl-1H-pyrazol-3-yloxy]ethyl}morpholine,
1-(3,4-Dichlorophenyl)-5-phenyl-3-[2-(pyrrolidin-1-yl)ethoxy]-1H-pyrazole,
1-(3,4-Dichlorophenyl)-5-phenyl-3-[3-(pyrrolidin-1-yl)propoxy]-1H-pyrazole,
1-{2-[1-(3,4-Dichlorophenyl)-5-phenyl-1H-pyrazol-3-yloxy]ethyl}piperidine,
1-{2-[1-(3,4-Dichlorophenyl)-5-phenyl-1H-pyrazol-3-yloxy]ethyl}-1H-imidazole,
2-{2-[1-(3,4-dichlorophenyl)-5-phenyl-1H-pyrazol-3-yloxy]ethyl}-1,2,3,4-tetrahydroisoquinoline,
4-{4-[1-(3,4-Dichlorophenyl)-5-methyl-1H-pyrazol-3-yloxy]butyl}morpholine,
1-(3,4-Dichlorophenyl)-5-methyl-3-[4-(pyrrolidin-1-yl)butoxy]-1H-pyrazole,
1-{4-[1-(3,4-Dichlorophenyl)-5-methyl-1H-pyrazol-3-yloxy]butyl}piperidine,
1-{4-[1-(3,4-Dichlorophenyl)-5-methyl-1H-pyrazol-3-yloxy]butyl}-4-methylpiperazine,
1-{4-[1-(3,4-Dichlorophenyl)-5-methyl-1H-pyrazol-3-yloxy]butyl}-1H-imidazole,
4-[1-(3,4-Dichlorophenyl)-5-methyl-1H-pyrazol-3-yloxy]-N,N-diethylbutan-1-amine,
1-{4-[1-(3,4-dichlorophenyl)-5-methyl-1H-pyrazol-3-yloxy]butyl}-4-phenylpiperidine,
1-{4-[1-(3,4-dichlorophenyl)-5-methyl-1H-pyrazol-3-yloxy]butyl}-6,7-dihydro-1H-indol-4(5H)-one,
2-{4-[1-(3,4-dichlorophenyl)-5-methyl-1H-pyrazol-3-yloxy]butyl}-1,2,3,4-tetrahydroisoquinoline,
4-{2-[1-(3,4-dichlorophenyl)-5-isopropyl-1H-pyrazol-3-yloxy]ethyl}morpholine,
2-[1-(3,4-Dichlorophenyl)-5-isopropyl-1H-pyrazol-3-yloxy]-N,N-diethylethanamine,
1-(3,4-Dichlorophenyl)-5-isopropyl-3-[2-(pyrrolidin-1-yl)ethoxy]-1H-pyrazole,
1-(3,4-Dichlorophenyl)-5-isopropyl-3-[3-(pyrrolidin-1-yl)propoxy]-1H-pyrazole,
1-{2-[1-(3,4-Dichlorophenyl)-5-isopropyl-1H-pyrazol-3-yloxy]ethyl}piperidine,
2-{2-[1-(3,4-dichlorophenyl)-5-isopropyl-1H-pyrazol-3-yloxy]ethyl}-1,2,3,4-tetrahydroisoquinoline,
4-{2-[1-(3,4-dichlorophenyl)-1H-pyrazol-3-yloxy]ethyl}morpholine,
2-[1-(3,4-dichlorophenyl)-1H-pyrazol-3-yloxy] N,N-diethylethanamine,
1-(3,4-dichlorophenyl)-3-[2-(pyrrolidin-1-yl)ethoxy]-1H-pyrazole,
1-{2-[1-(3,4-dichlorophenyl)-1H-pyrazol-3-yloxy]ethyl}piperidine,
1-(3,4-dichlorophenyl)-3-[3-(pyrrolidin-1-yl)propoxy]-1H-pyrazole,
1-{2-[1-(3,4-Dichlorophenyl)-5-methyl-1H-pyrazol-3-yloxy]ethyl}piperazine,
1-{2-[1-(3,4-Dichlorophenyl)-5-methyl-1H-pyrazol-3-yloxy]ethyl}pyrrolidin-3-amine,
4-{2-[1-(3,4-Dichlorophenyl)-4,5-dimethyl-1H-pyrazol-3-yloxy]ethyl}morpholine,
2-[1-(3,4-Dichlorophenyl)-4,5-dimethyl-1H-pyrazol-3-yloxy]-N,N-diethylethanamine,
1-(3,4-Dichlorophenyl)-4,5-dimethyl-3-[2-(pyrrolidin-1-yl)ethoxy]-1H-pyrazole,
1-(3,4-Dichlorophenyl)-4,5-dimethyl-3-[3-(pyrrolidin-1-yl)propoxy]-1H-pyrazole,
1-{2-[1-(3,4-Dichlorophenyl)-4,5-dimethyl-1H-pyrazol-3-yloxy]ethyl}piperidine,
4-{4-[1-(3,4-dichlorophenyl)-1H-pyrazol-3-yloxy]butyl}morpholine,
(2S,6R)-4-{4-[1-(3,4-dichlorophenyl)-1H-pyrazol-3-yloxy]butyl}-2,6-dimethylmorpholine,
1-{4-[1-(3,4-Dichlorophenyl)-1H-pyrazol-3-yloxy]butyl}piperidine,
1-(3,4-Dichlorophenyl)-3-[4-(pyrrolidin-1-yl)butoxy]-1H-pyrazole,
4-[1-(3,4-dichlorophenyl)-1H-pyrazol-3-yloxy]-N,N-diethylbutan-1-amine,
N-benzyl-4-[1-(3,4-dichlorophenyl)-1H-pyrazol-3-yloxy]-N-methylbutan-1-amine,
4-[1-(3,4-dichlorophenyl)-1H-pyrazol-3-yloxy]-N-(2-methoxyethyl)-N-methylbutan-1-amine,
4-{4-[1-(3,4-dichlorophenyl)-1H-pyrazol-3-yloxy]butyl}thiomorpholine,
1-[1-(3,4-Dichlorophenyl)-5-methyl-3-(2-morpholinoethoxy)-1H-pyrazol-4-yl]ethanone,
1-{1-(3,4-dichlorophenyl)-5-methyl-3-[2-(pyrrolidin-1-yl)ethoxy]-1H-pyrazol-4-yl}ethanone,
1-{1-(3,4-dichlorophenyl)-5-methyl-3-[2-(piperidin-1-yl)ethoxy]-1H-pyrazol-4-yl}ethanone,
1-{1-(3,4-dichlorophenyl)-3-[2-(diethylamino)ethoxy]-5-methyl-1H-pyrazol-4-yl}ethanone,
4-{2-[5-Methyl-1-(naphthalen-2-yl)-1H-pyrazol-3-yloxy]ethyl}morpholine, N,N-Diethyl-2-[5-methyl-1-(naphthalen-2-yl)-1H-pyrazol-3-yloxy]ethanamine,
1-{2-[5-Methyl-1-(naphthalen-2-yl)-1H-pyrazol-3-yloxy]ethyl}piperidine, and
5-Methyl-1-(naphthalen-2-yl)-3-[2-(pyrrolidin-1-yl)ethoxy]-1H-pyrazole,
or a pharmaceutically acceptable salt, prodrug or solvate thereof.

10. Sigma ligand according to any of claims 4 to 9, wherein the sigma ligand is 4-{2-[5-methyl-1-(naphthalen-2-yl)-1H-pyrazol-3-yloxy]ethyl} morpholine, or a pharmaceutically acceptable salt, isomer, prodrug or solvate thereof.

11. Sigma ligand according to claim 10, wherein the sigma ligand is 4-{2-[5-methyl-1-(naphthalen-2-yl)-1H-pyrazol-3-yloxy]ethyl} morpholine hydrochloride.

12. Use of sigma ligand as defined in any of claims 1 to 11 for the manufacture of a medicament for the prevention and/or treatment of bonce cancer pain.

13. A method of treatment of a patient suffering from bone cancer pain, or likely to suffer pain as a result of bone cancer, which comprises administering to the patient in need of such a treatment or prophylaxis a therapeutically effective amount of a sigma ligand as defined in any of claims 1 to 11.

14. A combination of at least one sigma ligand as defined in any of claims 1 to 11 and at least one opioid or opiate compound for simultaneous, separate or sequential administration, for use in the prevention and/or treatment of pain associated to bone cancer.

15. A combination according to claim 14, wherein the opioid is morphine.
